# EUROPEAN PATENT APPLICATION

(11) **EP 3 744 849 A1**
(43) Date of publication of application: **02.12.2020**
(21) Application number: 19382445.5
(22) Date of filing: 31.05.2019
(51) Int. Cl.: C12N 15/82

(54) **METHOD FOR MODULATING THE GENE SILENCING DEGREE INDUCED BY TRANS-ACTING SMALL INTERFERING RNAS**

(71) Applicant: Consejo Superior de Investigaciones Científicas (CSIC), 28006 Madrid (ES); Universitat Politècnica de València, 46022 Valencia (ES)
(72) Inventor: CARBONELL OLIVARES, Alberto Tomas, 46022 Valencia (ES); DAROS ARNAU, Jose Antonio, 46022 Valencia (ES)
(74) Representative: Pons Ariño, Angel

(57) **Abstract**

The invention provides a method for fine-tuning the gene silencing degree induced by a synthetic trans-acting small interfering RNA (syn-tasiRNA) which comprises varying the insertion position of the sequence of the syn-tasiRNA in its *TAS1c* precursor, regarding the miR173 cleavage target site, in order to obtain a higher or lower amount of the syn-tasiRNA expressed and consequently a higher or lower silencing activity induced by the same.

SEQUENCE LISTING

<110>  Consejo Superior de Investigaciones Científicas (CSIC)
       Universitat Politècnica de València

<120>  METHOD FOR MODULATING THE GENE SILENCING DEGREE INDUCED BY
       TRANS-ACTING SMALL INTERFERING RNAs

<130>  EP1641.1449

<160>  5

<170>  PatentIn version 3.5

<210>  1
<211>  21
<212>  DNA
<213>  Artificial Sequence

<220>
<223>  syn-tasiRNA sequence against the FT target gene

<400>  1
ttggttataa aggaagaggc c                                                21


<210>  2
<211>  21
<212>  DNA
<213>  Artificial Sequence

<220>
<223>  syn-tasiRNA sequence against the CH42 target gene

<400>  2
ttaagtgtca cggaaatccc t                                                21


<210>  3
<211>  21
<212>  DNA
<213>  Artificial Sequence

<220>
<223>  syn-tasiRNA sequence against the TSWV target

<400>  3
tgtaagacgt gattgtgtcc t                                                21


<210>  4
<211>  406
<212>  DNA
<213>  Arabidopsis thaliana

<400>  4
aaacctaaac ctaaacggct aagcccgacg tcaaatacca aaaagagaaa aacaagagcg      60

ccgtcaagct ctgcaaatac gatctgtaag tccatcttaa cacgaaagtg agatgggttc     120

ttagatcatg ttccgccgtt agatcgagtc atggtcttgt ctcatagaaa ggtactttcg     180

tttacttctt ttgagtatcg agtagagcgt cgtctatagt tagtttgaga ttgcgtttgt     240

cagaagttag gttcaatgtc ccggtccaat tttcaccagc catgtgtcag tttcgttcct     300

tcccgtcctc ttctttgatt tcgttgggtt acggatgttt tcgagatgaa acagcattgt     360

tttgttgtga tttttctcta caagcgaata gaccatttat cggtgg                    406

<210>  5
<211>  22
<212>  DNA
<213>  Artificial Sequence

<220>
<223>  miR173 cleavage target site

<400>  5
gtgatttttc tctacaagcg aa                                               22

## Description

The invention belongs to the fields of plant genetic engineering and artificial small interfering RNA-mediated gene silencing. Specifically, it relates to the field of the fine regulation of gene expression in plants by artificial or synthetic trans-acting small interfering RNAs (syn-tasiRNAs). In particular, this invention provides a method for modulating the efficiency of gene silencing induced by syn-tasiRNAs in plants, through which the desired gene silencing degree over the target gene/s is achieved.

### BACKGROUND ART

Artificial small RNAs (sRNAs) are 21-nucleotide (nt) RNAs designed to selectively and effectively silence transcripts with complementary sequences. Typically, highly active artificial sRNAs accumulate *in vivo* to high levels, and possess a high degree of base-pairing with their cognate target RNA(s). In plants, they have been extensively used to silence genes of interest in gene function studies, and to generate antiviral resistance or other type of crop improvements.

*Trans*-acting small interfering RNAs (tasiRNAs) are a specialized class of plant sRNAs that act *in trans* in post-transcriptional RNA silencing pathways to silence target RNA transcripts with high sequence complementarity. Synthetic tasiRNAs (syn-tasiRNAs) are designed and used for sRNA-based, specific gene silencing or knockdown in plants. Syn-tasiRNAs are thus a particular class of artificial sRNAs produced through a refined multi-step processing of *TAS* transcripts.

tasiRNAs originate from *TAS* gene transcripts and their generation is triggered by a microRNA (miRNA) that targets the *TAS* transcript, resulting in the production of 21-nt sRNAs that are phased with respect to the miRNA cleavage site. *Arabidopsis thaliana* (Arabidopsis) has four families of *TAS* genes: miR173 triggers tasiRNA production from *TAS1* and *TAS2,* miR390 from *TAS3* and miR828 from *TAS4. TAS* transcripts are initially targeted and sliced by a specific miRNA/ARGONAUTE (AGO) complex, and one of the cleavage products is converted to double-stranded RNA (dsRNA) by RNA-dependent RNA polymerase 6 (RDR6). The resulting dsRNA is sequentially processed by DCL4 into 21-nt small interfering RNA (siRNA) duplexes in register with the miRNA-guided cleavage site.

Syn-tasiRNAs have been shown to target RNAs in Arabidopsis when produced from *TAS1a* (Felippes and Weigel, 2009, EMBO Rep, 10: 264-270), *TAS1c* (de la Luz Gutierrez-Nava et al., 2008, Plant Physiol, 147: 543-551), and *TAS3a* (Felippes and Weigel, 2009, EMBO Rep, 10: 264-270) transcripts or from gene fragments fused to an upstream miR173 target site (Felippes et al., 2012, Plant J, 70: 541-547).

The use of this technology has benefited from the recent development of new methods for designing and generating artificial sRNA constructs such as the P-SAMS web tool for the automated design of artificial sRNAs (Fahlgren N., et al., 2016, Bioinformatics, 32: 157-158), and a new generation of B/c vectors optimized for one-step cloning and high expression of artificial sRNAs in plants (Carbonell A., et al., 2014, Plant Physiol, 165: 15-29). These methodologies have been optimized for time and cost effectiveness, high-throughput applicability and gene silencing efficacy. However, effective methods for modulating or fine-tuning the activity of artificial sRNAs in order to obtain the desired silencing degree of the genes of interest have not been reported yet.

It would be convenient to be able to modulate the degree of silencing of the target gene/s in an organism in order to obtain individuals with the desired degree of silencing. In particular, when the complete silencing of a specific gene of interest is lethal for the subject, it is crucial to obtain an intermediate gene silencing degree which leads to an improved phenotype while maintaining the viability of the individual.

Regarding methods for modulating the gene silencing efficiency of sRNAs, WO9853083 describes constructs and methods for enhancing the inhibition of a target gene within an organism which involve inserting into the gene silencing vector an inverted repeat sequence of all or part of a polynucleotide region within the vector. WO9953050 describes that intron containing hairpin dsRNA (ihpRNA) increases the efficiency of silencing.

EP1490489 describes methods and means for modulating the degree of silencing of a target gene in a eukaryotic cell, by including into the single initial dsRNA molecule provided to the eukaryotic cell, in addition to the target dsRNA inducing the silencing of the target gene, an amount of dsRNA sequences unrelated to the target dsRNA in a proportion reflecting the desired modulation of the degree of silencing of the target gene.

WO2007048629 provides methods and compositions for modulating RNA silencing efficiency by providing selective RISC (RNA-induced silencing complex) formation.

The introduction of mismatches and chemical modifications at the 3'end of siRNA sequences have also been proposed in order to attempt to reduce their silencing effect (Makiko Hamada, et al., 2002, Antisense and nucleic acid drug development, 12: 301-309).

Furthermore, it has been disclosed that varying the nucleic acid composition of siRNA molecules dramatically affects the duration and degree of gene silencing (Janelle S. Lamberton and Allen T. Christian, 2003, Molecular Biotechnology, 24: 111-119).

However, alternative methods for fine-tuning the degree of silencing induced by artificial trans-acting small interfering RNAs on a specific target gene are still required within the field of plant biotechnology. These methods would allow to obtain the desired silencing degree of the genes of interest in each particular case, leading to viable individuals that show the improved trait. These methods would further make possible to obtain several individuals showing different degrees of silencing of the same target gene, which is interesting, for instance, for genes involved in the flowering time. This would provide varieties of the same plant whose flowering occurs in different seasons, ensuring the production throughout the year.

### DESCRIPTION OF THE INVENTION

Inventors have surprisingly found that a different gene silencing efficiency/degree is achieved depending on the position in which the sequence of the synthetic *trans-*acting small interfering RNAs (syn-tasiRNA) is located within their *TAS1c* precursor relative to the miR173 cleavage site. Therefore, this invention solves the problem described above through a method for modulating the degree of gene silencing induced by a syn-tasiRNA, which comprises varying the insertion position of the sequence of the syn-tasiRNA in its *TAS1c* precursor, regarding the miR173 cleavage target site, in order to obtain a higher or lower accumulation (amount) of the syn-tasiRNA expressed and consequently a higher or lower silencing activity induced by the same.

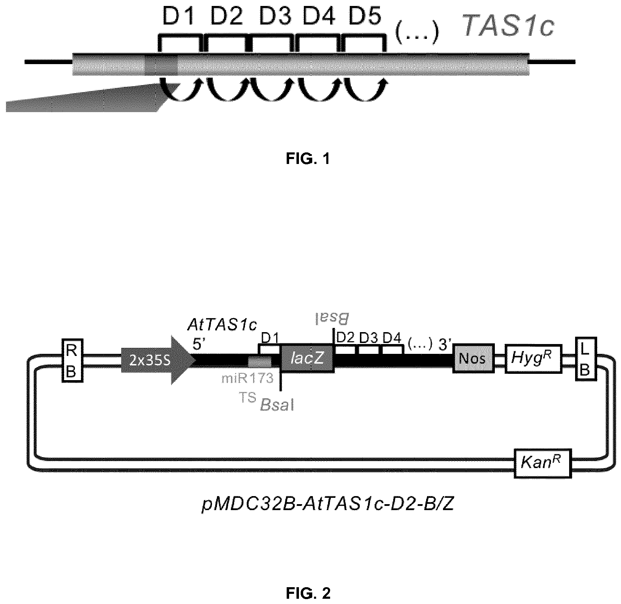

It is shown herein that the distance between the insertion position of the syn-tasiRNA sequence and the miR173 cleavage site in the *TAS1c* precursor is a particularly important factor that affects the syn-tasiRNA accumulation level in the cell expressing it. Accordingly, the syn-tasiRNA accumulation level in the cell is lower when its sequence is expressed from a position far away from the miR173 cleavage site, for instance when its sequence is expressed from positions D4 or D5, preferably D5, of the *TAS1c* transcript (see Figure 1 for positions arrangement). In this latter case, due to the lower syn-tasiRNA accumulation level, the gene silencing degree induced by this syn-tasiRNA over the target gene is also lower than the gene silencing degree induced by this same syn-tasiRNA expressed from the commonly used D3 position or from other positions nearer the miR173 cleavage site, such as D2.

The effect on syn-tasiRNA-mediated gene silencing of altering syn-tasiRNA accumulation as a result of modifying the syn-tasiRNA sequence position in its *TAS1c* precursor is shown in three examples described below through the expression of syn-tasiRNAs against *FLOWERING LOCUS T (FT)* and *CHLORINA 42* (*CH42*) Arabidopsis transcripts or against *Tomato spotted wilt virus* (TSWV) RNAs.

Syn-tasiRNA-mediated gene silencing can be modulated or fine-tuned by the method proposed in this invention, which opens the possibility of using artificial sRNAs to achieve different degrees of gene suppression. This is particularly attractive when genes, whose complete silencing leads to a lethal or toxic result, need to be partially silenced in order to obtain a desired trait, or to partially repress a gene or group of genes in an economically important crop in order to improve a particular trait and increase the market value of the corresponding product.

Thus, one aspect of the invention refers to a method for modulating or fine-tuning the gene silencing degree, activity or efficiency induced by a synthetic *trans*-acting small interfering RNA (syn-tasiRNA) over at least one target gene in a subject, wherein said method comprises the following steps:
a. providing a *TAS1c* transcript,
b. selecting the insertion position of the syn-tasiRNA sequence within the transcript of step (a), preferably among positions D2, D3, D4 or D5 (see Figure 1), depending on the gene silencing degree desired, wherein said positions are relative to the miR173 cleavage target site in said transcript, and placing the syn-tasiRNA sequence in the selected position, and
c. expressing the recombinant transcript obtained in step (b) in the cells of the subject,
wherein when a high gene silencing degree is desired, the sequence of the syn-tasiRNA is inserted (placed) near the miR173 cleavage target site, preferably at positions D2 or D3, more preferably D2, and
when lower gene silencing degrees are desired, the sequence of the syn-tasiRNA is inserted (placed) far away from the miR173 cleavage target site, preferably at positions D4 or D5, more preferably D5.

In another aspect, the present invention refers to a method, hereinafter "the method of the invention", for modulating or fine-tuning the gene silencing degree, activity or efficiency induced by a syn-tasiRNA over at least one target gene in a subject, wherein said method comprises the following steps:
a. providing a *TAS1c* transcript,
b. inserting the sequence of the syn-tasiRNA at a position near the miR173 cleavage target site in said transcript, preferably at position D2, when a high gene silencing degree is desired, or inserting the sequence of the syn-tasiRNA at a position far away from the miR173 cleavage target site in said transcript, preferably at positions D4 or D5, more preferably D5, when lower gene silencing degrees are desired 4, and
c. expressing the recombinant transcript obtained in step (b) in the cells of the subject.

This method of the invention may be applied to several *TAS* transcripts, preferably to Arabidopsis *TAS* transcripts. Thus, the *TAS1c* transcript provided in step (a) may be substituted by a *TAS* transcript, preferably an Arabidopsis *TAS* transcript, selected form the list consisting of: *TAS1a, TAS1b, TAS2, TAS3a, TAS3b, TAS3c* or *TAS4.* When the *TAS* transcript used in step (a) is *TAS1a, TAS1b, TAS1c* or *TAS2,* the positions referred to in step (b) are relative to the miR173 cleavage target site in said transcripts. When the *TAS* transcript used in step (a) is *TAS3a, TAS3b* or *TAS3c,* the positions referred to in step (b) are relative to the miR390 cleavage target site in said transcript. When the *TAS* transcript used in step (a) is *TAS4,* the positions referred to in step (b) are relative to the miR828 cleavage target site in said transcript.

A "*trans*-acting small interfering RNA", "*trans*-acting siRNA", "ta-siRNA", "tasiRNA" or "tasiR" is a class of small interfering RNA (siRNA) that acts *in trans* repressing gene expression through post-transcriptional gene silencing in plants. TasiRNAs are generated from non-coding *TAS* transcripts (precursors) through Argonaute (AGO)-mediated miRNA-guided cleavage followed by conversion to double stranded RNA (dsRNA) by the enzyme RNA-dependent RNA polymerase 6 (RDR6). The resulting dsRNA is further processed by Dicer-like enzyme 4 (DCL4) to produce a phased array of 21-nt siRNAs from positions adjoining the miRNA cleavage site. In particular, *TAS1c* transcripts undergo an initial AGO1 mediated cleavage at the 5' end that is guided by miR173. RDR6 then converts the transcript into a dsRNA fragment which then gets processed by DCL4 to generate the 21-nt tasiRNAs with 2 nucleotide 3' overhangs that target complementary mRNAs in *trans* (Figure 1).

There are four families of tasiRNA-generating loci (*TAS* genes) in Arabidopsis. *TAS1, TAS2,* and *TAS4* families require one miRNA binding site for cleavage to occur, while *TAS3* requires two binding sites. In addition to being present in Arabidopsis, evidence of tasiRNAs has also been found in the moss *Physcomitrella patens, Zea mays* (maize), *Oryza sativa* (rice), and other plants.

The "*TAS1c* transcript", "*TAS1c* precursor" or "*TAS1c*-based *trans*-acting small interfering RNA transcript" referred to in the present invention is a non-coding genetic transcript that comprises a cleavage target site for the microR173 located upstream (5') from the tasiRNA-generating consecutive (adjacent) positions (Figure 1). Said consecutive positions have 21 nt in length each and are thus suitable for the insertion of a syn-tasiRNA sequence of 21 nt. Preferably, the *TAS1c* transcript used in the present invention is the *TAS1c* transcript from Arabidopsis (also named *AtTAS1c*), more preferably it is the transcript identified in the GenBank under the accession number AY922999.1 which is shown in the SEQ ID NO: 4. The Arabidopsis *TAS1c* transcript is located at locus AT2G39675 of its genome.

"microR173" or "miR173" is a 22-nt miRNA with a 5' U that associates with AGO1 protein to guide the cleavage of *TAS1c* transcripts. A "miR173 cleavage target site" preferably comprises the sequence SEQ ID NO: 5.

The arrangement of the consecutive positions (D2, D3, D4, D5, D6, etc., preferably D2, D4, D5), relative to the miR173 cleavage target site, for the insertion of a syn-tasiRNA sequence in the *TAS1c* transcript referred to in this invention, is indicated in Figure 1. Each of these positions may comprise and generate a tasiRNA of 21-nt in length. These positions are adjacent to each other, which means that there are no further elements between one position and the next one, and are located downstream from the miR173 cleavage target site.

A "position near the miR173 cleavage target site" means any position located upstream the D3 position in the *TAS* transcript, preferably D2.

A "position far away from the miR173 cleavage target site" means any position located downstream the D3 position in the *TAS* transcript, preferably D4 or D5, more preferably D5.

The *TAS1c* transcript may be optionally inserted in a nucleic acid unit or construct, preferably a plasmid, that comprises said *TAS1c* transcript, in which one or more syn-tasiRNA sequence/s against one or more mRNAs of the gene/s of interest can be introduced to be cleaved, processed by RDR6 and DCL4 and expressed in a cell, as well as additional genetic elements that aid in the expression of the final syn-tasiRNA sequence. The additional elements comprised within this construct (e.g. constitutive Cauliflower mosaic virus 35S promoter and *Agrobacterium tumefaciens* nopaline synthase NOS terminator) are "operationally joined together", i.e. sequentially oriented therebetween in such a manner as to allow them to function in the intended manner, allowing that the generation and expression of the syn-tasiRNA sequence/s take place in compatible conditions with the other elements or sequences present in the construct.

Thus, in another preferred embodiment, the *TAS1c* transcript of step (a) is comprised, inserted or integrated in a suitable expression or cloning vector, more preferably in the *pMDC32B-AtTAS1c-D2-B*/*Z* plasmid described in Figure 2, in order to be expressed in the interior of the cells of the subject. An "expression or cloning vector" relates to a nucleic acid cassette wherein the *TAS1c* transcript can be integrated without affecting the viability and capacities of the vector. The expression vector is designed to direct the processing and expression of said *TAS1c* transcript in the interior of a target cell. In addition, the vector may comprise other genetic elements sequentially oriented (operationally linked) in such a manner that the *TAS1c* transcript may be processed and expressed in the cell. Said genetic elements may be, but not limited to, a promoter, a terminator, a leader sequence, a transcription initiation site, one or more replication origins, an untranslated regulatory region 3' and/or 5', a potentiator, a polyadenylation signal or any other control sequence, a reporter gene (for instance *lacZ* or a functional fragment thereof), enzyme restriction target site/s, antibiotic resistance gene/s, and the like. The expression vector may also include sequences that direct the expression of the *TAS1c* transcript comprised therein in a specific cell type, in a specific cellular sublocation or in a specific tissue.

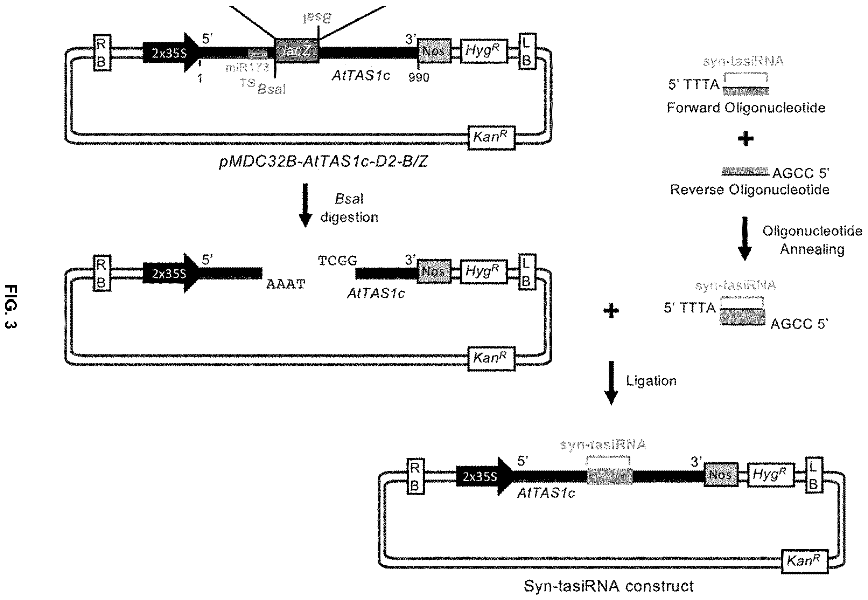

The *pMDC32B-AtTAS1c-D2-B*/*Z* plasmid is a plant expression vector containing a truncated *TAS1c* precursor sequence in which a *lacZ* fragment flanked by two inverted *Bsa*I sites was introduced between positions 3'D1[+] and 3'D2[+] (Figure 2). Generation of syn-tasiRNAs from this plasmid is illustrated in Figure 3 and is as follows: after *Bsa*I digestion, the *pMDC32B-AtTAS1c-D2-B*/*Z* vector has 5'-TTTA and 5'-CCGA ends, which are incompatible, thus preventing vector self-ligation. Syn-tasiRNA inserts are generated by annealing two partially overlapping oligonucleotides and are directionally ligated into the *Bsa*I-digested vector, and introduced in *E. coli.* Importantly, colony selection in plates is done in the presence of X-gal. Non-linearized plasmid molecules with no syn-tasiRNA insert propagate in *E. coli* as blue colonies while newly generated plasmids including syn-tasiRNA inserts propagate as white colonies.

This *pMDC32B-AtTAS1c-D2-B*/*Z* plasmid, as shown in Figure 2, was designed to introduce syn-tasiRNA sequences at position D2 (3'D2[+]), however, it may also be used to introduce syn-tasiRNA sequences at other positions if the *lacZ* fragment flanked by the two restriction sites, preferably inverted *Bsa*I sites, is introduced at other position/s of interest from which the syn-tasiRNA sequence/s are to be expressed.

The sequence of the "synthetic" ("syn-") tasiRNA referred to in the present invention is an artificially generated sequence with enough complementarity with the target mRNA of the gene that need to be silenced so as to induce the silencing of the same. Preferably, it shows a degree of complementarity with its target mRNA of at least 60%, preferably of at least 70% and more preferably of at least 80%, 90%, 91%, 92%, 93%, 94%, 95%, 96%, 97%, 98%, 99% or 100%, even more preferably 100%.

Methods for designing and generating synthetic tasiRNA sequences are well known in the art. For instance, they may be artificially obtained by means of conventional cloning and selection methods, or by sequencing, or using the P-SAMS web tool (Fahlgren N., et al., 2016, Bioinformatics, 32: 157-158). P-SAMS designs artificial tasiRNA sequences that have: (i) an AGO1-preferred 5'U nucleotide; (ii) a C in position 19 to produce a star strand with an AGO1 non-preferred 5'G, thus limiting the competition for AGO1 association with the guide strand; and (iii) an intentional mismatch with the target transcript at position 21 to reduce transitivity. P-SAMS outputs the sequence of the designed artificial tasiRNA together with the sequence of the two oligonucleotides required for cloning it into compatible vectors such as *AtTAS1c*-based B/c vectors (Carbonell A., et al., 2014, Plant Physiol, 165: 15-29). To clone P-SAMS designed artificial tasiRNA into the *pMDC32B-AtTAS1c-D2-B*/*Z* plasmid described above, forward and reverse oligos generated by P-SAMS must contain 5' TTTA and 5' CCGA instead of 5' TGTA and 5' AATG terminal nucleotides, respectively.

In the present invention, "modulating or fine-tuning the gene silencing degree, activity or efficiency" refers to fine-tune or regulate the gene silencing levels induced by a syn-tasiRNA by provoking varying degrees of silencing over the target gene.

A "high gene silencing degree" refers to a gene silencing activity or level induced by the syn-tasiRNA which is higher than the gene silencing activity or level induced by a syn-tasiRNA expressed from the commonly used D3 position in the *TAS1c* precursor/transcript.

A "low gene silencing degree" refers to a gene silencing activity or level induced by the syn-tasiRNA which is lower than the gene silencing activity or level induced by a syn-tasiRNA expressed from the commonly used D3 position in the *TAS1c* precursor/transcript.

In another preferred embodiment of the method of the invention, this method is used for simultaneously modulating the gene silencing degree of two different target genes by two different syn-tasiRNAs, wherein said method comprises, in step (b), inserting two different sequences of the syn-tasiRNAs at different positions of the transcript of step (a) depending on the gene silencing degree desired for each target gene. In a more preferred embodiment, the sequences of the two syn-tasiRNAs are not at positions D3 and D4 simultaneously.

In another preferred embodiment, the subject in which the method of the invention is to be applied is a eukaryotic organism, such as an animal including fish, avian animals, nematodes, insects, shellfish, mollusks, crustaceans, arthropods, mammals such as humans, or yeasts, fungi, molds, or a plant. More preferably, the subject to which this invention refers is a plant. Examples of plants are, without limitations, moss (*Physcomitrella patens*), potato, corn, cotton, Arabidopsis, *Nicotiana,* wheat, rice (*Oryza sativa*), sugar cane, sugarbeet, oilseed rape, maize vegetables, soybeans, tobacco, trees, etc.

In a particular embodiment, the plant is selected from the list consisting of any economically important crop and/or model plant such as cotton (*Gossypium hirsutum*), potato (*Solanum tuberosum*), corn (*Zea mays*), wheat (*Triticum aestivum*), rice (*Oryza sativa*), sugar cane (*Saccharum officinarum*), oilseed rape (*Brassica napus*), *Arabidopsis thaliana, Nicotiana benthamiana,* sugarbeet (*Beta vulgaris*), tobacco (*Nicotiana tabacum*) and soybean (*Glycine max*). In the most preferred embodiment, the plant is *Arabidopsis thaliana or Nicotiana benthamiana.*

In another preferred embodiment of the method of the invention, the syn-tasiRNA is a syn-tasiRNA against a target gene selected from the list consisting of: *FT, CH42,* TSWV, or any other gene whose controlled silencing could lead to an improved trait.

*FT* is the Flowering Locus T gene, *CH42* is the Chlorine 42 gene and TSWV is the Tomato spotted wilt virus.

The preferred syn-tasiRNA sequence against the *FT* target gene is SEQ ID NO: 1.

The preferred syn-tasiRNA sequence against the *CH42* target gene is SEQ ID NO: 2.

The preferred syn-tasiRNA sequence against the TSWV target is SEQ ID NO: 3.

In a particular embodiment of the method of the invention, the subject is an *Arabidopsis thaliana* plant and the target gene against which the syn-tasiRNA is directed is *FT* or *CH42.*

In another particular embodiment of the method of the invention, the subject is a *Nicotiana benthamiana* plant and the target against which the syn-tasiRNA is directed is TSWV.

Another aspect of the invention refers to a *TAS1c* transcript that comprises a syn-tasiRNA sequence inserted at position D2, D3, D4 or D5, preferably at position D2 or D5, wherein said position is relative to the miR173 cleavage target site. This transcript will be also referred to herein as "the transcript of the invention".

In a preferred embodiment of the transcript of the invention, the syn-tasiRNA is a syn-tasiRNA against a target gene selected from the list consisting of: *FT, CH42,* TSWV, or any other gene whose controlled silencing could lead to an improved trait. Another aspect of the invention refers to a cell, hereinafter "the cell of the invention", preferably a plant cell, or a plant, comprising the transcript of the invention.

The cell of the invention has been transduced, transfected, transformed or similar with the transcript of the invention using any genetic engineering technique from those known in the art for such purpose. Said techniques may be, for example, but not limited to, floral dipping method using *Agrobacterium tumefaciens* GV3101 strain, injection or microinjection, *ex vivo* transformation, electroporation, precipitation with calcium phosphate, DEAE-dextrane followed by polyethylene glycol, sonication, biolistics, viral infection, liposome-mediated transfection (lipofection) or via receptor, or other techniques widely known in the state of the art.

In a preferred embodiment, the plant cell is from Arabidopsis or *Nicotiana* or the plant is Arabidopsis or *Nicotiana.* More preferably, *Nicotiana* is *Nicotiana benthamiana.*

In a more preferred embodiment, the plant or plant cell is from Arabidopsis and it comprises the transcript of the invention, wherein the syn-tasiRNA is a syn-tasiRNA against the target gene *FT* or *CH42.*

In another preferred embodiment, the plant or plant cell is from *Nicotiana,* more preferably *Nicotiana benthamiana,* and it comprises the transcript of the invention, wherein the syn-tasiRNA is a syn-tasiRNA against the target TSWV.

Unless otherwise defined, all technical and scientific terms used herein have the same meaning as that commonly understood by one of ordinary skills in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practicing the invention. The following examples and drawings are provided by way of illustration and are not intended to be limiting of the present invention.

### DESCRIPTION OF THE DRAWINGS

**Fig. 1****. Diagram of the Arabidopsis *TAS1c* transcript.** miR173 cleavage target site is indicated with a dark box. Only twenty-one-nucleotide tasiRNA-generating positions D1 to D5 are indicated for simplicity.
**Fig. 2****. Diagram of the *pMDC32B-AtTAS1c-D2-B*/*Z* vector for cloning and expressing syn-tasiRNAs.** This vector was designed to introduce syn-tasiRNA sequences at position 3'D2[+].
**Fig. 3****. Diagram of the steps for generating syn-tasiRNA constructs using *pMDC32B-AtTAS1c-D2-B*/*Z* vector.** The syn-tasiRNA insert obtained after annealing the two overlapping oligonucleotides has 5'-ATTA and 5'-GTTC overhangs and is directly inserted into the *Bsa*I-linearized *pMDC32B-AtTAS1c-D2-B*/*Z* vector to generate the syn-tasiRNA construct.

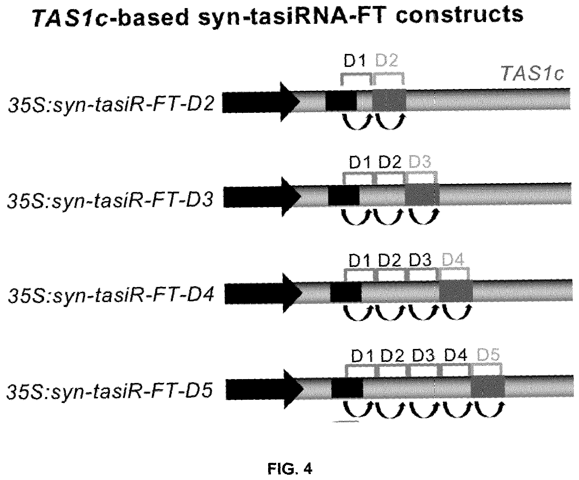

**Fig. 4****. Diagram of the constructs used to express a syn-tasiRNA against *FT* from different *TAS1c* precursor positions.** The light grey box represents the syn-tasiRNA sequence inserted in the construct in different positions each time. The dark grey box represents the target site for miR173, where the miR173 binds to. The curved arrows show the movement of the DCL4 when dicing, while the straight arrows show where the miR173 or the syn-tasiRNAs cut their targets.

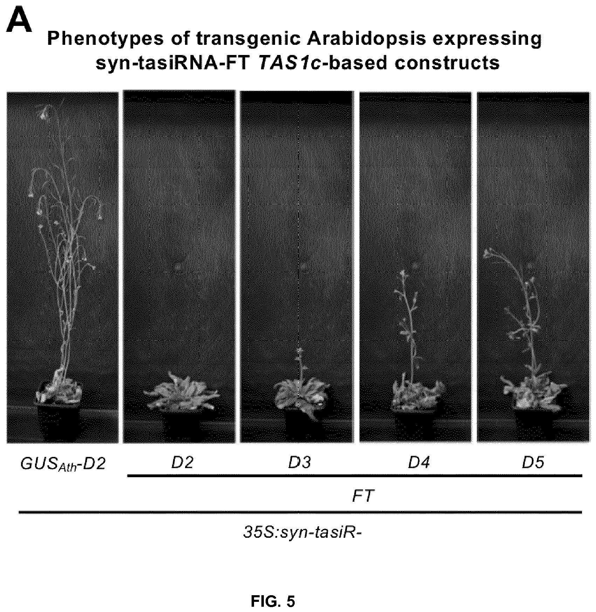

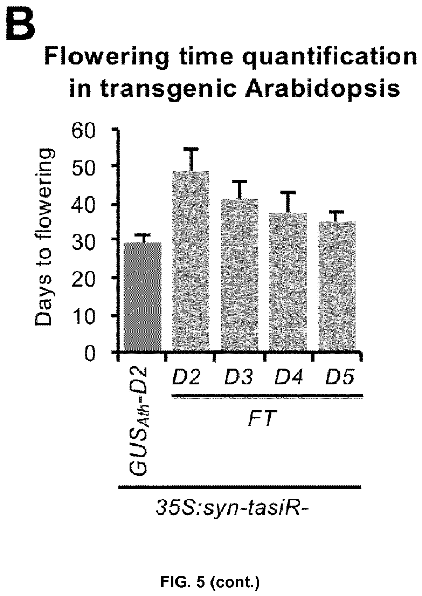

**Fig. 5****. Phenotype analysis of transgenic Arabidopsis expressing a syn-tasiRNA against *FT* from different *TAS1c* precursor positions. A)** Pictures of 41 days-old plants. Each picture includes a representative plant expressing a specific construct. **B)** Bar graph showing the average time needed to flower for each type of transgenic Arabidopsis. GUS_{Ath}-D2 is the control line. D2 to D5 are the four different types of transgenics expressing syn-tasiR-FT. Error bars indicate standard deviation.

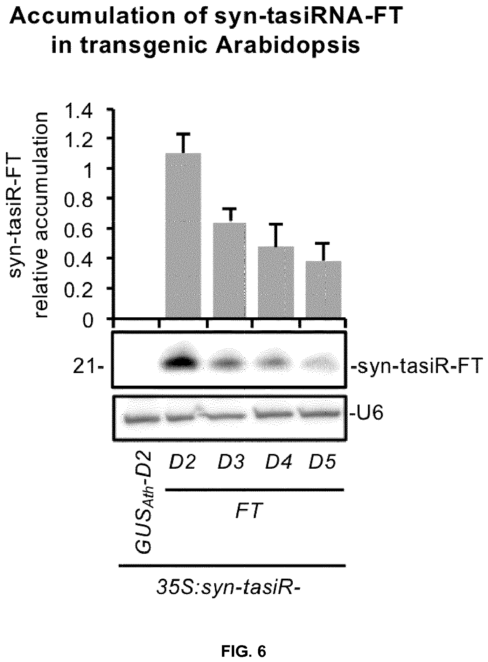

**Fig. 6****. Northern blot analysis of syn-tasiR-FT accumulation from different Arabidopsis transgenic plants.** Bar graph shows the relative mean accumulation of syn-tasiR-FT calculated from three biological replicates for each type of transformed Arabidopsis. Each biological replicate is a pool of 9-12 independent lines. U6 blot is shown as loading control. Error bars indicate standard deviation.

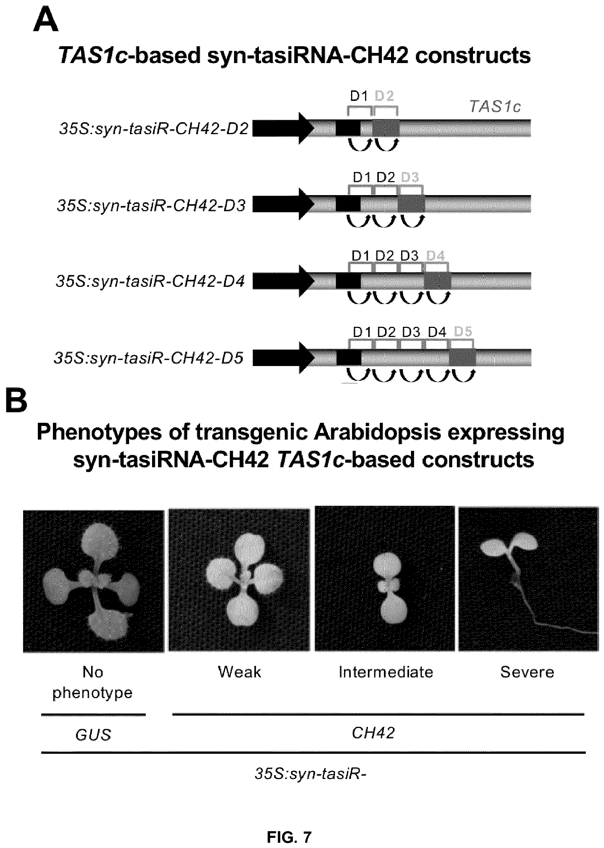

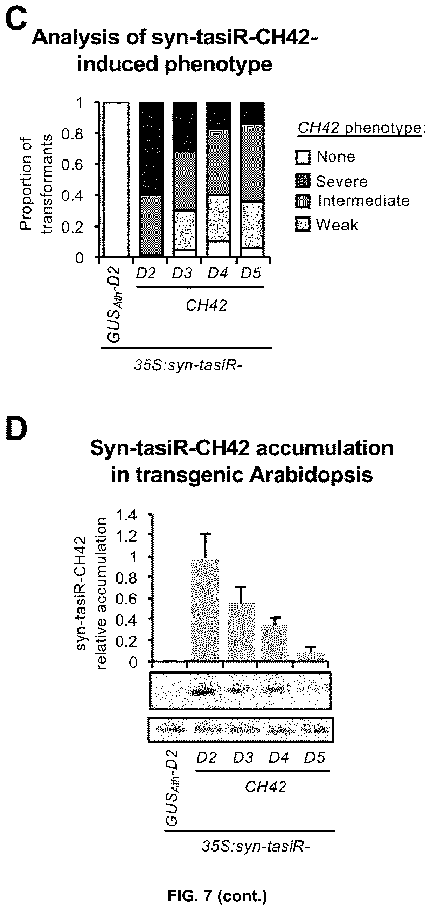

**Fig. 7****. Fine-tune regulation of *CH42* in Arabidopsis. A)** Diagram of the constructs used to express a syn-tasiRNA against *CH42* from different *TAS1c* precursor positions. Other details are as in Figure 4. **B)** Bleaching phenotypes of 10-day-old transgenic Arabidopsis seedlings expressing syn-tasiR-Ch42 *TAS1c*-based constructs. **C)** Quantification of syn-tasiR-CH42-induced phenotypes in 10 day-old transgenic seedlings. Bar graphs show the proportion of transformants showing none, severe, intermediate or weak phenotype. **D)** Northern blot analysis of syn-tasiR-CH42 accumulation from different Arabidopsis transgenic plants. Bar graph shows the relative mean accumulation of syn-tasiR-CH42 calculated from three biological replicates for each type of transformed Arabidopsis. Other details are as in Figure 6.

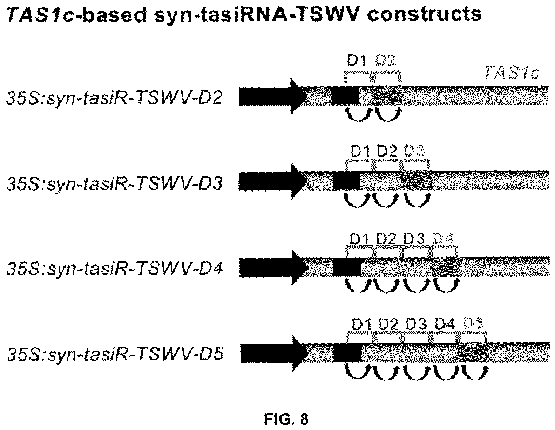

**Fig. 8****. Diagram of the constructs used to analyze the effect of the position of the syn-tasiR-TSWV in its precursor.** The light grey box represents the syn-tasiRNA sequence inserted in the construct in different positions each time. The dark grey colored box represents the target site for miR173.

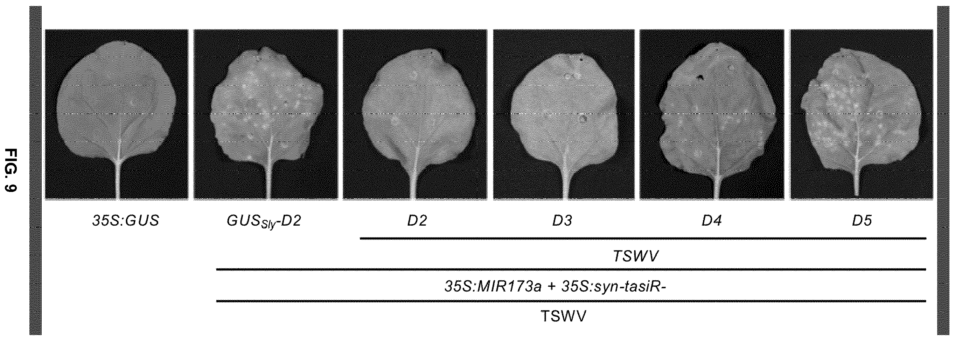

**Fig. 9****. Analysis at 5 days post-inoculation of TSWV-induced symptoms in inoculated *N. benthamiana* leaves expressing syn-tasiR-TSWV from different *TAS1c* positions.**

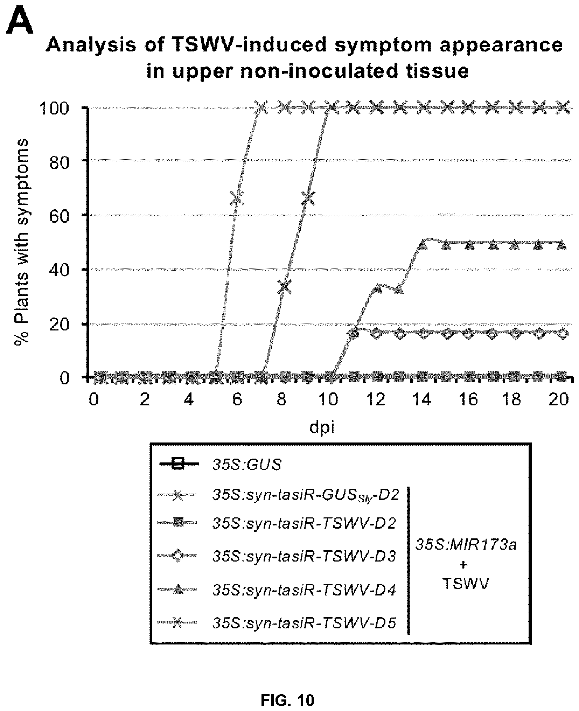

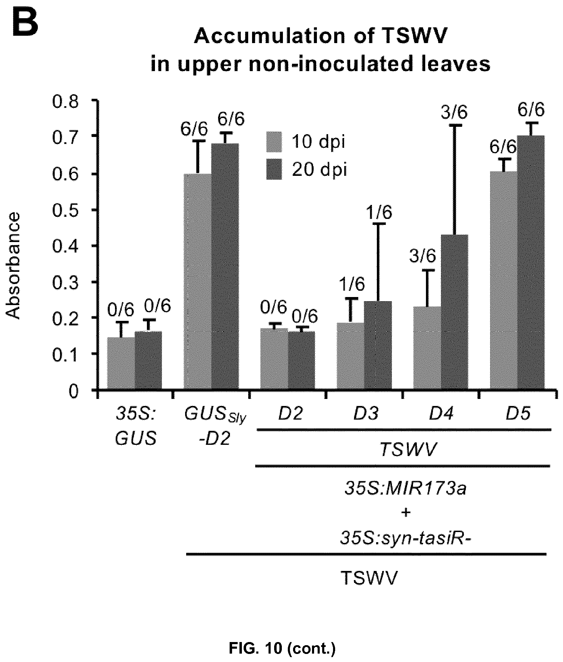

**Fig. 10****. Symptom and accumulation analyses of TSWV in *N. benthamiana* bioassay. A)** Graph showing the amount of symptomatic plants with the past of the days. **B)** ELISA assay showing the accumulation of the virus at 10 and 20 days post-inoculation.

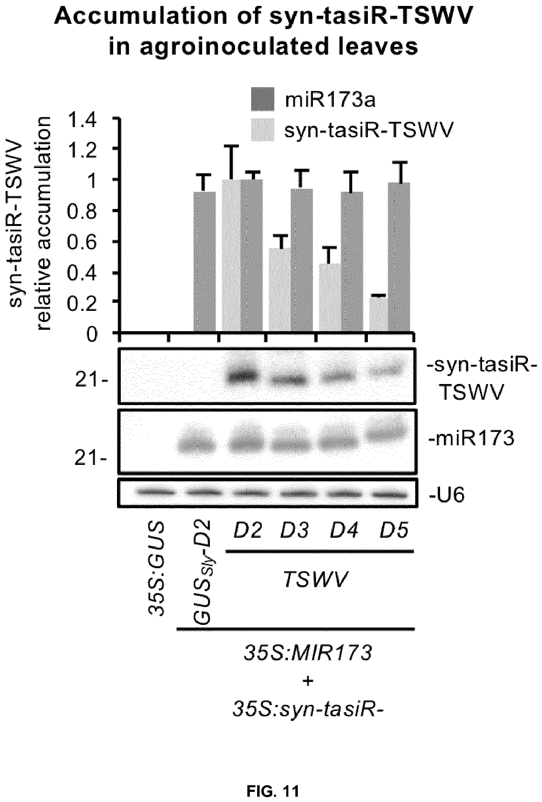

**Fig. 11****. syn-tasiR-TSWV accumulation in *N. benthamiana* leaves.** Bar graph showing the accumulation of syn-tasiRNA-TSWV and miR173 in leaves at 2 days postagroinfiltration.

### EXAMPLE

It was hypothesized herein whether it could be possible to modulate the level of expression of syn-tasiRNAs, and their subsequent silencing efficiency, through their differential positioning in their corresponding precursor. Moreover, it was tested if syn-tasiRNAs could be successfully expressed from position D2 of *TAS1c* precursors, instead of from the commonly used D3 position, a matter that was not analyzed before.

Therefore, several assays were performed in order to test if syn-tasiRNAs expressed from position D2 accumulate more than those expressed from position D3, and those expressed from D3 more than those from D4, and those from D4 more than those from D5.

In order to test this hypothesis, the accumulation level and silencing efficiency of syn-tasiRNAs expressed from positions D2, D3, D4 or D5 against Arabidopsis *FT* or *CH42* mRNAs or against TSWV mRNAs in *N. benthamiana* were analyzed, as detailed below.

### Example 1. Fine-tune regulation of FT expression in Arabidopsis.

To test the reliability of this system on an endogenous gene, the *FT* gene whose silencing is relatively easy to quantify by recording time of flowering was selected. The more silenced the *FT* gene is, the higher the flowering time of this individual is. Moreover, a 21-nt sequence was shown to induce high levels of *FT* silencing (SEQ ID NO: 1). As it can be seen on Figure 4, four different constructs expressing this syn-tasiRNA 21-nt sequence from positions D2, D3, D4 or D5 of *TAS1c* precursor (constructs *35S:syn-tasiR-FT-D2, 35S:syn-tasiR-FT-D3, 35S:syn-tasiR-FT-D4* and *35S:syn-tasiR-FT-D5,* respectively) were built according to the generation scheme shown in Figure 3. A control construct, *35S:syn-tasiR-GUSAth-D2,* was also generated which expresses, from D2 position, a syn-tasiRNA against the *GUS* gene from *E. coli* designed to avoid off-targets in Arabidopsis. This control syn-tasiRNA should not have any effect on *FT* expression.

Arabidopsis plants were transformed independently with each construct following the floral dipping method using *Agrobacterium tumefaciens* GV3101 strain. Approximately 50,000 seeds of each independent transformation were sterilized in a sterile hood. Transgenic Arabidopsis were selected by growing seeds in plates containing MS salts, bacto-agar and hygromycin among other reagents. After seed plating, plates were kept at 4°C in the dark for 2 days before being transferred to a growth chamber, prior to soil transfer.

Forty-eight to ninety-six T1 independent lines per construct were transferred to soil and analyzed. For each individual, the time of flowering (days from seed plating to first opened flower) was recorded. Results showed in Figure 5 show an increase on the number of days needed to flower the nearer the syn-tasiRNA is expressed from the miR173 target site in the *TAS1c* precursor.

Once phenotyped, a molecular analysis by small RNA northern blot was carried out in order to verify the accumulation levels of syn-tasiR-FT from the different types of transgenic Arabidopsis. This corresponds to Figure 6, where it can be observed a decrease in the accumulation of syn-tasiR-FT the farther from position D1 is expressed. These results explain the phenotype data, as plants with higher syn-tasiR-FT accumulation have more delayed flowering and viceversa. This is consistent with the initial hypothesis, since *FT* helps moving into the reproductive phase, which means that the more silenced *FT* is, the more time the plants will need to enter their reproductive phase. The U6 blot showed on the figure is the control used for verifying that the different levels of accumulation were not influenced by the amount of total RNA used in the analysis, since U6 is an RNA sequence expressed endogenously by the plant and not affected by syn-tasiR-FT expression.

### Example 2. Fine-tune regulation of CH42 expression in Arabidopsis.

Similar results were obtained for fine-tune regulating *CH42* expression in Arabidopsis. CH42 magnesium chelatase silencing leads to a bleached phenotype with loss of green color. Briefly, a 21-nt sequence (syn-tasiR-CH42) was shown to induce high levels of *CH42* silencing (SEQ ID NO: 2). As before, four different constructs expressing syn-tasiR-CH42 from positions D2, D3, D4 or D5 of *TAS1c* precursor (constructs *35S:syn-tasiR-CH42-D2, 35S:syn-tasiR-CH42-D3, 35S:syn-tasiR-CH42-D4* and *35S:syn-tasiR-CH42-D5,* respectively) were built (Figure 7A). As before, the control construct *35S:syn-tasiR-GUSAth-D2* used should not have any effect on *CH42* expression.

Forty-eight to ninety-six T1 independent lines per construct were transferred to soil and analyzed. The phenotype of 10 days-old seedlings was recorded and included in one of the following four categories: no phenotype (wild-type green plants), weak (slightly pale plants), moderate or intermediate (plants with two true leaves of yellow color) and severe (no visible true leaves, yellow cotyledons) (Figure 7B). Results showed in Figure 7C show an increase of the bleached phenotype the nearer the syn-tasiRNA is expressed from the miR173 target site in the *TAS1c* precursor.

Small RNA northern blot analysis showed a decrease in the accumulation of syn-tasiR-CH42 the farther from position D1 is expressed (Figure 7D). Again, these results explain the phenotype data, as plants with higher syn-tasiR-CH42 accumulation have a stronger bleached phenotype and viceversa.

### Example 3. Modulation of TSWV infectivity in N. benthamiana.

Same approach as in the case of the *FT* and CH42 genes was followed. In this case, a syn-tasiR-TSWV with high antiviral activity (SEQ ID NO: 3) was expressed from positions D2, D3, D4 or D5 of *TAS1c* (constructs *35S:syn-tasiR-TSWV-D2, 35S:syn-tasiR-TSWV-D3, 35S:syn-tasiR-TSWV-D4* and *35S:syn-tasiR-TSWV-D5,* respectively; see Figure 8). The control construct *35S:syn-tasiR-GUSSly-D2* expressing a syn-tasiRNA from D2 against *E. coli GUS* gene with no off-targets in tomato (TSWV natural host) was also analyzed.

Each construct was co-expressed in one leaf from each six independent *N. benthamiana* plants together with a construct expressing miR173 (since it is required to trigger syn-tasiRNA biogenesis from *TAS1c* precursors and *N. benthamiana* does not produce it by its own). Two days later this same agroinfiltrated leaf was inoculated with TSWV. The idea was to let the plants sufficient time (2 days) to produce enough amount of the syn-tasiR-TSWV before the virus is inoculated, as described before (Carbonell et al., 2019, Mol Plant Microbe Interact., 32: 142-156.). Plants were monitored for symptom expression during 20 days. If the syn-tasiRNA worked properly, the accumulation of the virus should be lower in the first days compared to the control and, therefore, the systemic spread of the virus should take longer (or should even not take place). If the hypothesis was right, the closer to position D1 syn-tasiR-TSWV was expressed, the higher the delay in infection should be.

At 5 days post-inoculation (dpi) of the virus, it can be seen a difference between the different inoculated leaves from different plants: those expressing syn-tasiR-TSWV in position D2 and D3 showed no necrotic lesions typical of TSWV local infection, while control plants expressing syn-tasiR-GUSSly or those expressing syn-tasiR-TSWV from D4 or D5 exhibited similar level of lesions (Figure 9).

At the end of the experiment, 20 dpi, it was observed that all control plants showed systemic symptoms, while none of the plants expressing syn-tasiR-TSWV from D2 did. Moreover, 1 out of 6, 3 out of 6 and 6 out of 6 plants expressing syn-tasiR-TSWV from D3, D4 or D5, respectively, showed systemic symptoms (Figure 10A). These differences were highlighted when we plotted for each construct the percentage of plants that showed symptoms through each of the days of the bioassay (Figure 10A). The analysis of such graph shows that between 6-7 dpi all control plants displayed symptoms, while plants expressing *35S:syn-tasiR-TSWV-D5* become all infected with a delay of 2-3 days in symptom appearance compared to controls. Half of the plants expressing *35S:syntasiR-TSWV-D4* became symptomatic, but with a considerable delay of 5-9 days compared to control plants. Virus accumulation in all plants of the experiment was analyzed by ELISA. Results from analysis at 10 and 20 dpi are shown in Figure 10B, and corroborate the results obtained from phenotype analysis. Taken together, these results indicate that the farther from miR173 target site syn-tasiR-TSWV is expressed the less antiviral effect it induces.

Finally, syn-tasiR-TSWV accumulation was analyzed in agroinfiltrated leaves at 2 days postagroinfiltration (dpa). As observed in Figure 11, syn-tasiR-TSWV decreases the farther it is expressed from the miR173 site. These results are consistent with the phenotype and TSWV accumulation analyses in terms that the antiviral activity of the syn-tasiR-TSWV gradually decreases the farther from the miR173 target site it is expressed. Moreover, these results are also consistent with those obtained with syn-tasiR-FT and syn-tasiR-CH42 in Arabidopsis. Importantly, miR173 expression was similar across all samples indicating that the expression of syn-tasiR-TSWV was not influenced by different levels of expression of this miRNA, necessary for the initial processing of the *TAS1c* transcript.

## Claims

1. A method for modulating the gene silencing degree induced by a synthetic *trans-*acting small interfering RNA over at least one target gene in a subject, wherein said method comprises the following steps:
a. providing a *TAS1c* transcript,
b. inserting the sequence of the synthetic trans-acting small interfering RNA at a position near the miR173 cleavage target site in the transcript of step (a) when a high gene silencing degree is desired, or inserting the sequence of the synthetic *trans*-acting small interfering RNA at a position far away from the miR173 cleavage target site in the transcript of step (a) when lower gene silencing degrees are desired, and
c. expressing the transcript obtained in step (b) in the cells of the subject.

2. The method according to claim 1 for modulating the gene silencing degree of two different target genes by two different synthetic *trans*-acting small interfering RNAs, wherein said method comprises, in step (b), inserting two different sequences of the synthetic *trans*-acting small interfering RNAs at different positions of the transcript of step (a) depending on the gene silencing degree desired for each target gene.

3. The method according to any of claims 1 or 2, wherein the subject is a plant.

4. The method according to claim 3, wherein the plant is selected from the list consisting of cotton, potato, corn, wheat, rice, sugar cane, oilseed rape, *Arabidopsis thaliana, Nicotiana benthamiana,* sugarbeet, tobacco and soybean.

5. The method according to claim 4, wherein the plant is *Arabidopsis thaliana or Nicotiana benthamiana.*

6. The method according to any of claims 1 to 5, wherein the synthetic *trans*-acting small interfering RNA is a synthetic *trans*-acting small interfering RNA against a target gene selected from the list consisting of: *FT, CH42* or TSWV.

7. The method according to any of claims 1 to 6, wherein the subject is an *Arabidopsis thaliana* plant and the target gene is *FT* or *CH42.*

8. The method according to any of claims 1 to 6, wherein the subject is a *Nicotiana benthamiana* plant and the target is TSWV.

9. The method according to any of claims 1 to 8, wherein the position near the miR173 cleavage target site in the transcript is D2.

10. The method according to any of claims 1 to 9, wherein the position far away from the miR173 cleavage target site in the transcript is D4 or D5.

11. The method according to claim 10, wherein the position far away from the miR173 cleavage target site in the transcript is D5.
